# EUROPEAN PATENT APPLICATION

(11) **EP 0 741 188 A2**
(43) Date of publication of application: **06.11.1996**
(21) Application number: 96303133.1
(22) Date of filing: 03.05.1996
(51) Int. Cl.: C12N 15/17, C12N 5/10, C07K 14/62, A61K 38/28, A61K 48/00

(54) **Single chain insulin with high bioactivity**

(30) Priority: 05.05.1995 US 435762; 05.05.1995 US 435503
(71) Applicant: ELI LILLY AND COMPANY, Indianapolis, Indiana 46285 (US)
(72) Inventor: Chance, Ronald Eugene, Westfield, Indiana 46074 (US); Dimarchi, Richard Dennis, Carmel, Indiana 46033 (US); Hoffmann, James Arthur, Greenwood, Indiana 46143 (US); Long, Harlan Beall, Carmel, Indiana 46032 (US); Miller, Anne Reifel, Indianapolis, Indiana 46208 (US)
(74) Representative: Hudson, Christopher Mark

(57) **Abstract**

The instant invention provides polypeptide compounds of the formula b-BP-a having significant insulin activity, nucleotide sequences encoding said polypeptides, vectors comprising said nucleotide sequences, and cell lines transformed with said vectors useful for treating diabetes.

SEQUENCE LISTING

(1) GENERAL INFORMATION:

(i) APPLICANT: ELI LILLY AND COMPANY
(B) STREET: Lilly Corporate Center
(C) CITY: Indianapolis
(D) STATE: Indiana
(E) COUNTRY: United States of America
(F) ZIP: 46285

(ii) TITLE OF INVENTION: SINGLE CHAIN INSULIN WITH HIGH BIOACTIVITY

(iii) NUMBER OF SEQUENCES: 10

(iv) CORRESPONDENCE ADDRESS:
(A) ADDRESSEE: C. M. Hudson
(B) STREET: Erl Wood Manor
(C) CITY: Windlesham
(D) STATE: Surrey
(E) COUNTRY: United Kingdom
(F) ZIP: GU2[?] 6PH

(v) COMPUTER READABLE FORM:
(A) MEDIUM TYPE: Floppy disk
(B) COMPUTER: Macintosh
(C) OPERATING SYSTEM: Macintosh 7.0
(D) SOFTWARE: Microsoft Word 5.1

(vi) CURRENT APPLICATION DATA:
(A) APPLICATION NO: 96303133.1

(2) INFORMATION FOR SEQ ID NO: 1:

(i) SEQUENCE CHARACTERISTICS:
(A) LENGTH: 12 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO: 1:

Gln Pro Leu Ala Leu Glu Gly Ser Leu Gln Lys Arg
1               5                   10

(2) INFORMATION FOR SEQ ID NO: 2:

(i) SEQUENCE CHARACTERISTICS:
(A) LENGTH: 12 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO: 2:

Gly Tyr Gly Ser Ser Ser Arg Arg Ala Pro Gln Thr
1               5                   10

(2) INFORMATION FOR SEQ ID NO: 3:

(i) SEQUENCE CHARACTERISTICS:
(A) LENGTH: 63 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO: 3:

Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Gln Pro
            20                  25                  30

Leu Ala Leu Glu Gly Ser Leu Gln Lys Arg Gly Ile Val Glu Gln Cys
        35                  40                  45

Cys Thr Ser Ile Cys Ser Leu Tyr Gln Leu Glu Asn Tyr Cys Asn
    50                  55                  60

(2) INFORMATION FOR SEQ ID NO: 4:

(i) SEQUENCE CHARACTERISTICS:
(A) LENGTH: 63 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO: 4:

Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Gly Tyr
            20                  25                  30

Gly Ser Ser Ser Arg Arg Ala Pro Gln Thr Gly Ile Val Glu Gln Cys
        35                  40                  45

Cys Thr Ser Ile Cys Ser Leu Tyr Gln Leu Glu Asn Tyr Cys Asn
    50                  55                  60

(2) INFORMATION FOR SEQ ID NO: 5:

(i) SEQUENCE CHARACTERISTICS:
(A) LENGTH: 201 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 5:

ATGCGTATGT TTGTTAACCA ACACCTGTGC GGCTCCCACC TGGTGGAAGC TCTGTACCTG
60

GTGTGCGGTG AACGTGGCTT CTTCTACACC CCGAAGACGC AGCCGCTGGC CCTCGAGGGT
120

TCCCTGCAGA AGCGTGGCAT TGTGGAACAA TGCTGTACCA GCATCTGCTC CCTGTACCAG
180

CTGGAGAACT ACTGCAACTA G
201

(2) INFORMATION FOR SEQ ID NO: 6:

(i) SEQUENCE CHARACTERISTICS:
(A) LENGTH: 66 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO: 6:

Met Arg Met Phe Val Asn Gln His Leu Cys Gly Ser His Leu Val Glu
1               5                   10                  15

Ala Leu Tyr Leu Val Cys Gly Glu Arg Gly Phe Phe Tyr Thr Pro Lys
            20                  25                  30

Thr Gln Pro Leu Ala Leu Glu Gly Ser Leu Gln Lys Arg Gly Ile Val
        35                  40                  45

Glu Gln Cys Cys Thr Ser Ile Cys Ser Leu Tyr Gln Leu Glu Asn Tyr
    50                  55                  60

Cys Asn
65

(2) INFORMATION FOR SEQ ID NO: 7:

(i) SEQUENCE CHARACTERISTICS:
(A) LENGTH: 264 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 7:

ATGGCCCTGT GGATGCGCCT CCTGCCCCTG CTGGCGCTGC TGGCCCTGTG GGGACCTGAC
60

CCAGCCGCAG CCTTTGTTAA CCAACACCTG TGCGGCTCCC ACCTGGTGGA AGCTCTGTAC
120

CTGGTGTGCG GTGAACGTGG CTTCTTCTAC ACCCCGAAGA CGCAGCCGCT GGCCCTCGAG
180

GGTTCCCTGC AGAAGCGTGG CATTGTGGAA CAATGCTGTA CCAGCATCTG CTCCCTGTAC
240

CAGCTGGAGA ACTACTGCAA CTAG
264

(2) INFORMATION FOR SEQ ID NO: 8:

(i) SEQUENCE CHARACTERISTICS:
(A) LENGTH: 87 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO: 8:

Met Ala Leu Trp Met Arg Leu Leu Pro Leu Leu Ala Leu Leu Ala Leu
1               5                   10                  15

Trp Gly Pro Asp Pro Ala Ala Ala Phe Val Asn Gln His Leu Cys Gly
            20                  25                  30

Ser His Leu Val Glu Ala Leu Tyr Leu Val Cys Gly Glu Arg Gly Phe
        35                  40                  45

Phe Tyr Thr Pro Lys Thr Gln Pro Leu Ala Leu Glu Gly Ser Leu Gln
    50                  55                  60

Lys Arg Gly Ile Val Glu Gln Cys Cys Thr Ser Ile Cys Ser Leu Tyr
65                  70                  75                  80

Gln Leu Glu Asn Tyr Cys Asn
                85

(2) INFORMATION FOR SEQ ID NO: 9:

(i) SEQUENCE CHARACTERISTICS:
(A) LENGTH: 115 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 9:

AGCTTCCACC ATGGCCCTGT GGATGCGCCT CCTGCCCCTG CTGGCGCTGC TGGCCCTGTG
60

GGGACCTGAC CCAGCCGCAG CCTTTGTTAA CCAACACCTG TGCGGCTCCC ACCTG
115

(2) INFORMATION FOR SEQ ID NO: 10:

(i) SEQUENCE CHARACTERISTICS:
(A) LENGTH: 159 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 10:

GTGGAAGCTC TGTACCTGGT GTGCGGTGAA CGTGGCTTCT TCTACACCCC GAAGACGCAG
60

CCGCTGGCCC TCGAGGGTTC CCTGCAGAAG CGTGGCATTG TGGAACAATG CTGTACCAGC
120

ATCTGCTCCC TGTACCAGCT GGAGAACTAC TGCAACTAG
159

## Description

The present invention relates to single-chain peptides having insulin activity useful for treatment of diabetes, to nucleotide compounds encoding said peptides, to vectors comprising said nucleotides compounds, and to cells transformed with said vectors.

Although elimination or modification of the naturally occurring connecting peptide (or C-chain) of proinsulin may be expected to increase insulin activity relative to native proinsulin, none of these previously modified C-chain proinsulins have demonstrated the level of activity of the compounds of the present invention. The proinsulin structure has previously been modified by incorporation of shortened C-chain fragments. However, these so called "mini-C" proinsulins have not demonstrated insulin activity greater than approximately 2% of the native insulin activity.

The art suggests that a 2-chain insulin construction is preferred over a single-chain insulin construction. Yu and co-workers describe a "clipped" proinsulin molecule to form a two-chain construction. *Biological Activity of Proinsulin and Related Polypeptides in the Fat Tissue* J. Biol. Chem. 248:3753-3761 (1973). The authors demonstrate that an increase in insulin activity results from making a two-chain construct, suggesting that full insulin potency requires a two-chain molecule. Similarly, Peavy et al., demonstrate that clipped (2-chain) versions of proinsulin analogs possess insulin activity of less than 20%, but possess greater insulin activity than natural single-chain proinsulin molecules. J. Biol. Chem. 260:13989-13994 (1985). Markussen, et al. (International Journal of Peptide and Protein Research 26:70-77 (1985)) describe a molecule wherein the carboxyl terminus of the des(b30) insulin B-chain is linked to the amino terminus of the A-chain. In later work, this analog was determined to have a three dimensional crystal structure essentially identical with that of insulin, yet surprisingly retained essentially no insulin activity. Derewenda, U. et al., J. Mol. Biol (1991) 220:425-433.

Single-chain proinsulin molecules having shortened C-chains have failed to demonstrate sufficient insulin activity to qualify as potential therapeutics. Wetzel, et al. [Gene 16:63-71 (1981)] describe a "mini-C" proinsulin having a C-chain of six amino acids. This molecule is described as an insulin precursor which may be cleaved to form a two-chain insulin molecule. There is no discussion of the biological activity of the mini-C proinsulin described in this reference. Furthermore, there is no suggestion that this molecule would have any insulin activity at all. It is merely being used as a precursor for generating insulin. The estimated biological insulin activity is approximately one-tenth of one percent (0.1%) that of insulin. Kubiak and Cowburn (Biochemical Journal (1986) 234:665-670) showed a construction with an insulin B-chain of 22 amino acids, an insulin A-chain of 21 amino acids and the full proinsulin C-chain. This molecule again demonstrated approximately 0.02% insulin potency, even less than that of natural sequence proinsulin. Hoechst, A.G., European Patent Publication No. 0347781 A2, demonstrates a mini-C proinsulin containing the A and B chains of insulin joined by a single arginine residue. There is no biological activity data presented in this publication, despite the statement that this is useful for treating diabetes as a substitute for human proinsulin. This reference is directed to the construction of an insulin precursor which may be cleaved to create natural sequence insulin from recombinant sources similar to the Wetzel et al. reference. Similarly Kobayashi, et al. (Diabetes Research and Clinical Practice 7:25-28 (1989)) demonstrate a proinsulin molecule with a shortened C-chain of the construction b(1-29)-Ala-Ala-Lys-a(1-21) proinsulin. This molecule has approximately 0.2% of the activity of human insulin. This reference again further demonstrates that mini-C proinsulin molecules having shortened C-chains, especially approximately 3 amino acids, have very low insulin activity and would not be suitable as an insulin replacement.

Therefore, the art demonstrates that proinsulin molecules having shortened C-chains possess very little insulin activity. Similarly, proinsulin molecules having full length C-chains, i.e., approximately 35 amino acids have very low insulin activity, usually only approximately 2% of insulin activity (note the data presented for hPI in a, Table 1, herein). Therefore, one would expect that a proinsulin-like molecule having a shortened C-chain or a proinsulin-like molecule which has not been clipped, i.e., a single-chain insulin construct, would not be suitable as a therapeutic replacement for insulin.

Industrial production of insulin from recombinantly-expressed, single-chain insulin precursors involves specific enzymatic cleavages. These enzymes add significant production costs for raw materials, process control, and purification. A single-chain peptide with signifcant insulin activity obviates these enzyme-related production costs and represents an advancement in the art.

Similarly, mammalian insulin synthesis involves cleavages of a single-chain precursor catalyzed by highly specific, endogenous enzymes found only in pancreatic beta cells. A major challenge for gene therapy for treating diabetes is to provide for expression of the insulin precursor in cells having the specific endogenous enzyme activities required for proper maturation of the molecule. A solution to this challenge is a single-chain peptide with significant insulin activity. Such a single-chain peptide can be expressed in cells lacking the specific endogenous enzymes, thereby increasing dramatically the types of host cells useful for gene therapy of diabetes.

The instant invention provides a single-chain polypeptide compound of the formula:

b-BP-a (1)

wherein
b is the B-chain of insulin or a functional analog thereof,
a is the A-chain of insulin or a functional analog thereof,
BP is a Bridging Peptide.

The instant invention provides gene therapy methods and constructions allowing for the in vivo production under glucose-regulated control of a single-chain polypeptide compound of the Formula 1.

Nucleotide sequences encoding the compounds of Formula 1 are useful in the treatment of both Type I and Type II diabetes.

The present invention further provides recombinant nucleotide vectors useful in the treatment of diabetes incorporating a nucleotide sequence encoding a compound of Formula 1.

The present invention further provides recombinant host cells incorporating said vectors capable of secreting the compound of Formula 1 in response to glucose stimulus.

The present invention further provides a method of treating diabetes mellitus comprising the use of the recombinant nucleotide sequences encoding the compounds of Formula 1, vectors containing said nucleotide sequences, and host cells transformed with said vectors.

As previously indicated herein, the art provides a number of single-chain insulin compounds which fail to demonstrate significant or therapeutically useful levels of insulin activity. The following table provides comparative data of examples of the compounds of formula 1 in comparison to human proinsulin (hPI), human insulin, and IGF-1. Two different assay procedures were employed as Table 1 indicates in vitro data determined in accordance with the procedure of Example 5 herein and Table 2 provides in vivo rat bioassay data which was performed in substantial accordance with the teaching of Belagaje, et al. (United States Patent No. 5,304,473).

**Table 1**

| Construct¹ | %IGF-1 Activity² | %Insulin Activity² |
|---|---|---|
| a) hPI | <0.1 | 2.4 |
| b) des(31-53) hPI | 0.04 | 14 |
| c) bCa | 23 | 34 |
| d) human insulin | 0.19 | 100 |
| e) IGF-1 | 100 | .18 |

| | | |
|---|---|---|
| ¹ all constructs use the naturally occurring human peptide sequences | | |
| ² measured by procedure of Example 5 herein. | | |

**Table 2**

| Relative In Vivo Rat Bioassay Data on a Molar Basis | |
|---|---|
| human proinsulin | 16% |
| des(31-53)hPI | 62% |
| human insulin | 100% |

A comparison of the bioactivity of human proinsulin with that of native insulin demonstrates that the C-chain ("c") is a suppressor of insulin activity. As previously discussed, the art indicates that a two-chain insulin molecule possesses significantly greater levels of insulin activity than any single-chain construct, indicating that a single-chain construct possessing high insulin activity would be unlikely. Previous studies with mini-proinsulins have validated this concept where various connecting peptides have been used to link the insulin A and B-chains with marked reductions in insulin potency. The compounds of the present invention provide a single-chain insulin comprising the insulin A and B-chains resulting in a molecule which possess an unexpectedly, i.e. several fold, increases in both in vitro and in vivo insulin activities.

### Bridging Peptide:

A Bridging Peptide is defined for purposes of the present invention as an amino acid sequence of from about 10 to about 14 amino acids regardless of amino acid sequence. Furthermore, the insulin activity is not determined by the amino acid sequence of the Bridging Peptide. If one compares the two examples of the compounds prepared in accordance with the teaching of the examples, one will note the lack of any sequence homology between the Bridging Peptide of the compounds. For example the Bridging Peptide of the molecules described in Table 1 have the following amino acid sequences:
GLN-PRO-LEU-ALA-LEU-GLU-GLY-SER-LEU-GLN-LYS-ARG des(31-53)hPI
GLY-TYR-GLY-SER-SER-SER-ARG-ARG-ALA-PRO-GLN-THR bCa

However, as indicated in the results presented in Table 1, both of these compounds demonstrate significant insulin activity.

Preferably the Bridging Peptide should not contain amino acid sequences which interact with the IGF-1 receptor. Three residues (tyrosine and the tandem arginines) in the IGF-1 C-domain have been identified as being important for IGF-1 activity, i.e. interaction with the IGF-1 receptor.

Preferably there should be an absence of cleavage sites within the Bridging Peptide to retain the single-chain nature of the protein, especially to intracellular E.coli proteases as well as human serum proteases.

Preferably, the 12 amino acids are derived from an existing sequence of 12 residues from within the C-peptide of hPI so as to minimize potential immunogenicity, but is does not have to be any particular 12-mer from the sequence.

In the most preferred practice of the invention as exemplified herein, an amino acid sequence of 12 residues is derived from the sequence of amino acids 54-65 of the human proinsulin C-peptide.

### A Chain and B Chain Functional Analogs

The invention further provides functional analogs of the compounds of Formula 1. The term "functional analog" refers to a molecule having similar functional properties but a modified structure relative to the naturally occurring form of that molecule or compound. Functional analogs include fragments of (or additions to) the parent molecule having similar functional properties and reactivities as the parent molecule. Such functional analogs typically exhibit the same qualitative biological activity as the naturally-occurring peptide, although functional analogs may also be designed which significantly modify the characteristics of a peptide. Functional analogs are ordinarily engineered variations, but such functional analogs include naturally occurring allelic or interspecies variations of the predominant naturally occurring amino acid sequence.

Functional analogs of the compounds of formula 1 are generally created by modification of the amino acid sequence of insulin A or B chains in a specific and limited manner. While the site for introducing an amino acid sequence variation is predetermined, the mutation per se need not be predetermined. For example, in order to optimize the performance of a mutation at a given site, random mutagenesis may be conducted at the target codon or region and the resulting functional analogs screened for the optimal balance of desired activities.

Functional analogs of the peptides are typically generated by deletion, insertion, or substitutions of a single (or few) amino acid residues. Such modifications generally are made in accordance with the following Table 3:

**TABLE 3**

| Original Residue | Exemplary Substitutions |
|---|---|
| Ala | Ser |
| Arg | Lys |
| Asn | Gln, His |
| Asp | Glu |
| Cys | Ser |
| Gln | Asn |
| Glu | Asp |
| Gly | Pro |
| His | Asn, Gln |
| Ile | Leu, Val |
| Leu | Ile, Val |
| Lys | Arg, Gln, Glu |
| Met | Leu, Ile |
| Phe | Met, Leu, Tyr |
| Ser | Thr |
| Thr | Ser |
| Trp | Tyr |
| Tyr | Trp, Phe |
| Val | Ile, Leu |

Substantial changes in function and/or immunological identity may be achieved by selecting substitutions that are less conservative than those in Table 3, i.e., by selecting residues that differ more significantly in their effect on maintaining (a) secondary or tertiary structure of the polypeptide backbone, (b) the charge or hydrophobicity of the residue, or (c) the steric bulk of the amino acyl side chain. For example, substitutions which would ordinarily be expected to produce significant alterations in protein properties will be those in which (a) a hydrophilic residue is substituted by a hydrophobic residue; (b) a cysteine (involved in a disulfide linkage) or proline is substituted for any other residue; (c) a residue having a positively charged side chain is substituted for a negatively charged residue; or (d) a residue having a bulky side chain is substituted for a residue having a much smaller side chain.

A variety of amino acid modifications have been introduced into the primary structure of insulin insulin A and B chains resulting in insulin A-chain and B-chain functional analogs. These functional analogs have demonstrated a wide variety of desirable characteristics useful for treating various forms of diabetes, to facilitate commercial (especially recombinant) production, and/or to provide more desirable pharmaceutical formulations. A sample list of such modifications to the primary structure of insulin and proinsulin is provided in Table 4 below. The instant invention includes compounds of Formula 1 incorporating such known modifications to the structure of the insulin A- and B-chains

**Table 4**

| Insulin A-Chain and B-Chain Amino Acid Substitutions | | | | |
|---|---|---|---|---|
| Gly a₂₁ | Glu a₂₁ | Ser a₂₁ | Thr b₁₀ | Asp b₂₅ |
| Ser a₂₁ | Leu a₂₁ | Gly a₂₂ | Asp b₁₀ | His b₂₅ |
| Ala a₂₁ | Met a₂₁ | Ala a₂₂ | Arg b₁₀ | Glu b₂₆ |
| His a₂₁ | Tyr a₂₁ | Asp b₉ | Ile b₁₂ | Glu b₂₇ |
| Asp a₂₁ | Val a₂₁ | Asn b₉ | His b₁₆ | Asp b₂₈ |
| Thr a₂₁ | Ile a₂₁ | His b₉ | Gln b₁₇ | Ala b₃₀ |
| Gln a₂₁ | Trp a₂₁ | Glu b₁₀ | Gln b₂₀ | des-b₃₀ |
| Pro b₂₉ | Lys b₂₈ | Asn a₂₁-NH2 | | |

Furthermore, it will known in the art that the first four and last five amino acids of the B-chain of insulin are generally not essential for insulin activity. Consequently, functional analogs of the B-chain as defined in formula 1 emcompass deletions and/or substitutions of the amino and/or carboxyl terminal residues of the B-chain.

In the preferred practice of the invention, as exemplified herein, the A- and B-chains are derived from human insulin. However, other embodiments of this invention include compounds derived from rabbit, monkey, horse, rat I, rat II, porcine, bovine, lamb, dog, guinea pig, chinchilla, or duck insulin molecules. Other embodiments of this invention may be directed to functional analogs of the compounds of Formula 1 derived from the aforementioned species.

In the preferred practice of the invention as exemplified herein, a compound of the Formula 1 comprises the amino acid sequence:
PHE-VAL-ASN-GLN-HIS-LEU-CYS-GLY-SER-HIS-LEU-VAL-GLU-ALA-LEU-TYR-LEU-VAL-CYS-GLY-GLU-ARG-GLY-PHE-PHE-TYR-THR-PRO-LYS-THR-GLN-PRO-LEU-ALA-LEU-GLU-GLY-SER-LEU-GLN-LYS-ARG-GLY-ILE-VAL-GLU-GLN-CYS-CYS-THR-SER-ILE-CYS-SER-LEU-TYR-GLN-LEU-GLU-ASN-TYR-CYS-ASN (Sequence ID No. 1)

In the preferred practice of the invention as exemplified herein, another compound of the Formula 1 comprises the amino acid sequence:
PHE-VAL-ASN-GLN-HIS-LEU-CYS-GLY-SER-HIS-LEU-VAL-GLU-ALA-LEU-TYR-LEU-VAL-CYS-GLY-GLU-ARG-GLY-PHE-PHE-TYR-THR-PRO-LYS-THR-GLY-TYR-GLY-SER-SER-SER-ARG-ARG-ALA-PRO-GLN-THR-GLY-ILE-VAL-GLU-GLN-CYS-CYS-THR-SER-ILE-CYS-SER-LEU-TYR-GLN-LEU-GLU-ASN-TYR-CYS-ASN (Sequence ID No. 2)

### Synthesis:

The compounds of the present invention may be produced either by recombinant DNA technology or well known chemical procedures, such as solution or solid-phase peptide synthesis, or semi-synthesis in solution beginning with protein fragments coupled through conventional solution methods. The synthesis of the compounds of the present invention may proceed by solid-phase peptide synthesis or by recombinant methods, however recombinant methods are preferred if a high yield is desired.

### A. Solid Phase:

A compound of Sequence ID No. 2 was synthesized by solid-phase methodology utilizing an Applied Biosystems 430A peptide synthesizer (commercially available from Applied Biosystems, Foster City, California) using synthesis cycles supplied by Applied Biosystems in substantial accordance with the teaching of examples 1-5 herein.

### B. Recombinant DNA:

The compounds of Formula 1 may also be produced by recombinant methods. The basic steps in the recombinant production of insulin analogs of the formula 1 include:
a) constructing a synthetic or semi-synthetic DNA encoding the compound of the formula 1,
b) integrating said DNA into an expression vector in a manner suitable for the expression of the hybrid insulin analogs of the formula 1,
c) transforming an appropriate eukaryotic or prokaryotic host cell with said expression vector,
d) culturing said transformed or transfected host cell, and
e) recovering and purifying the recombinantly produced compound of the formula 1.

The coding sequence for recombinant preparation of the compounds of the formula 1 may be wholly synthetic, semi-synthetic or the result of modification of the native insulin cDNAs. DNA sequences designed for recombinant production insulin A and B chains and proinsulin are well known. United States patent No. 4,431,740 (issued February 14, 1984) describes recombinant production of human proinsulin. United States Patent No. 4,366,246 (issued December 28, 1982) describes DNA sequences encoding and the recombinant production of human insulin A-chain and B-chain. Synthetic genes, the in vitro or in vivo transcription and translation of which will result in the production of compounds of the Formula 1 may be constructed by techniques well known in the art. Owing to the natural degeneracy of the genetic code, the skilled artisan will recognize that a sizable yet definite number of DNA sequences may be constructed which encode the compounds of the Formula 1.

As exemplified herein a synthetic DNA gene of the sequence:
ATG CGT ATG TTT GTT AAC CAA CAC CTG TGC GGC TCC CAC CTG GTG GAA GCT CTG TAC CTG GTG TGC GGT GAA CGT GGC TTC TTC TAC ACC CCG AAG ACG CAG CCG CTG GCC CTC GAG GGT TCC CTG CAG AAG CGT GGC ATT GTG GAA CAA TGC TGT ACC AGC ATC TGC TCC CTG TAC CAG CTG GAG AAC TAC TGC AAC TAG (Sequence ID No. 3)
was used for the recombinant production of the compound of formula 1 having the amino acid sequence:
Met-Arg-Met-PHE-VAL-ASN-GLN-HIS-LEU-CYS-GLY-SER-HIS-LEU-VAL-GLU-ALA-LEU-TYR-LEU-VAL-CYS-GLY-GLU-ARG-GLY-PHE-PHE-TYR-THR-PRO-LYS-THR-GLN-PRO-LEU-ALA-LEU-GLU-GLY-SER-LEU-GLN-LYS-ARG-GLY-ILE-VAL-GLU-GLN-CYS-CYS-THR-SER-ILE-CYS-SER-LEU-TYR-GLN-LEU-GLU-ASN-TYR-CYS-ASN (Sequence ID No. 4).

The synthetic DNA sequence identified was prepared by techniques well known in the art in substantial accordance with the teachings of Brown, et al. (1979) Methods in Enzymology, Academic Press, N.Y., Vol. 68, pgs. 109-151. The DNA sequence was be generated using conventional DNA synthesizing apparatus such as an Applied Biosystems Model 380A or 380B DNA synthesizer (commercially available from Applied Biosystems, Foster City, California).

Furthermore, direct expression of DNA compounds including compounds of Formula 1 in procaryotic cells results in a compound possessing an amino terminal methionine residue. This Met may be removed by the action of cyanogen bromide (CNBr) where the particular compound does not otherwise possess an internal methionine residue. Alternatively, a Met-less compound may be obtained by engineering an introductory leader sequence which may be removed by limited proteolytic digestion (e.g. trypsin, diaminopeptidases such a Cathepsin C (U.S. Patent No. 5,126,249 issued June 30, 1992 and European Publication No. 0557076A1 published August 25, 1993), the diaminopeptidase of Dictyostelium discoideum (European Publication No. 0595476A2 published May 4, 1994) or enterokinase).

In the practice of the invention as exemplified herein, the compound of formula 1 identified as Sequence ID No. 1 was produced first as a Met-Arg-Met-des(31-53)hPI precursor (Sequence ID No. 4). The Met-Arg-Met amino terminal sequence is provided for the convenient removal of the characteristic amino terminal methionine resides of recombinant proteins through the use of CNBr in substantial accordance with the teaching of example 6 herein.

Construction of suitable vectors containing the desired coding and control sequences employ standard ligation techniques. Isolated plasmids or DNA fragments are cleaved, tailored, and religated in the form desired to form the plasmids required. To effect the translation of the compounds of formula 1, one inserts a DNA sequence encoding the compound into an appropriate recombinant DNA expression vector through the use of appropriate restriction endonucleases. The coding sequence is designed to possess restriction endonuclease cleavage sites at either end of the transcript to facilitate isolation from and integration into these amplification and expression plasmids. The coding sequence may be readily modified by the use of synthetic linkers to facilitate the incorporation of this sequence into the desired cloning vectors by techniques well known in the art. The particular endonucleases employed will be dictated by the restriction endonuclease cleavage pattern of the parent expression vector to be employed. The restriction sites are chosen so as to properly orient the coding sequence with control sequences to achieve proper in-frame reading and expression of compound of the Formula 1. Furthermore, the coding sequence is positioned so as to be properly aligned with the promoter and ribosome binding site of the expression vector, both of which are functional in the host cell in which the compound of the Formula 1 is to be expressed.

In general, plasmid vectors containing promoters and control sequences which are derived from species compatible with the host cell are used with these hosts. The vector ordinarily carries a replication site as well as marker sequences which are capable of providing phenotypic selection in transformed cells. For example, E. coli is typically transformed using pBR322, a plasmid derived from an E. coli species (Bolivar, et al., Gene 2: 95 [1977]). pBR322 contains genes for ampicillin and tetracycline resistance and thus provides easy means for identifying transformed cells. The pBR322 plasmid, or other microbial plasmids should also contain or be modified to contain promoters and other control elements commonly used in recombinant DNA construction.

In the preferred practice of the invention as exemplified herein, when the host cell is E. coli K12 RV308, the promoter-operator region is the lambda pL promoter operator region.

In the preferred practice of the invention as exemplified herein when the host cell is an E. coli K12 cell, the expression vector was pRB284 which contains a DNA sequence encoding the Met-Arg-Met-des(31-53)hPI. The parent vector to this plasmid is the vector pCZR126S may be prepared according to the teaching of Belagaje, et al. United States Patent No. 5,304,473 issued April 9, 1994, the entire teaching of which is herein incorporated by reference. As exemplified herein, the plasmid pCZR126S was digested with NdeI and BamHI to remove the bovine growth hormone gene encoded thereby. The plasmid pRB284 was constructed by inserting a double stranded DNA sequence encoding the Met-Arg-Met-des(31-53)hPI into the NdeI-BamHI site.

The techniques of transforming cells with the aforementioned vectors are well known in the art and may be found in such general references as Maniatis, et al. (1989) Molecular Cloning: A Laboratory Manual, Cold Spring Harbor Press, Cold Spring Harbor Laboratory, Cold Spring Harbor, New York or Current Protocols in Molecular Biology (1989) and supplements. In addition to prokaryotes, eukaryotic microbes such as yeast cultures may also be used. Saccharomyces cerevisiae, or common baker's yeast is the most commonly used eukaryotic microorganism, although a number of other strains are commonly available. For expression in Saccharomyces, the plasmid YRp7, for example, (ATCC-40053, Stinchcomb, et al., (1979) Nature 282:39; Kingsman et al., [1979] Gene 7:141 ; Tschemper et al., [1980] Gene 10:157) is commonly used. The methodology for transforming the E. coli cell lines employed in the most preferred practice of the invention may be obtained by reference to the Examples herein. The precise conditions under which the transformed E. coli cells are cultured is dependent on the nature of the E. coli host cell line and the expression or cloning vectors employed. For example, vectors which incorporate thermoinducible promoter-operator regions, such as the c1857 thermoinducible lambda-phage promoter-operator region, require a temperature shift in the culture conditions so as to induce protein synthesis.

As exemplified herein, the plasmid denoted pRB284 was used to transform E. coli K12 RV308 to facilitate recombinant production a bacterial E. coli environment. The transformed host cell is then cultured to a sufficient cell density at which time the temperature is shifted from 37°C to about 42°C to initiate transcription from the c1857 thermoinducible lambda-phage promoter-operator region.

The recombinantly produced protein is then isolated from the cell culture by well known purification procedures employing techniques such as centrifugation, ion exchange chromatography, size exclusion chromatography, RP-HPLC, etc. In the recombinant production of des(31-53)hPI as exemplified herein, the transformed E. coli host cells were harvested using centrifugation. The cells were lysed by the addition of lysozyme and the first stage isolation of the S-sulfonated form of Met-Arg-Met-des(31-53)hPI compound was achieved by size exclusion chromatography. The Met-Arg-Met amino terminal leader sequence was removed by the action of CNBr to yield the des(31-53)hPI. The S-sulfonates of des(31-53)hPI molecules were converted to the disulfide-paired, folded des(31-53)hPI molecules using a combination of high pH and added thiol in substantial accordance with the teaching of Frank, B.H., et al., (1981) in Peptides. Synthesis, Structure and Function. Proceedings of the Seventh American Peptide Symposium (Rich, D.H. and Gross, E. Eds.) pgs. 729-738, Pierce Chemical Co., Rockford, IL. Subsequent rounds of solvent exchange and purification were achieved using column chromatography in a denaturing environment. The proteins were characterized for purity and identity by amino acid analysis, and Fast Atom Bombardment Mass Spectrometry (FAB-MS) with the expected results.

### Gene Therapy

Many heritable diseases result from the absence of a functional gene necessary to provide the animal with an adequate supply of a necessary protein. Gene therapy attempts to replace or supply a functional gene encoding the protein using recombinant DNA or RNA vectors. Although there are a number of methods used to incorporate the desired DNA into the target cells, there are two primary procedures employed termed ex vivo and in vivo therapy for the introduction of the gene therapy nucleotide sequence into the target animal.

Ex vivo therapy consists of four primary steps (1) the target cells are removed from the individual animal in need of therapy, (2) the gene therapy nucleotide sequence is incorporated into the target cell in vitro, (3) identification, isolation, and expansion of the transformed cells expressing the protein of interest and (4) replacement of the protein expressing cells into the animal. Ex vivo therapy generally results in the incorporation of the nucleotide sequence encoding the protein of interest into the chromosomal DNA of the target cell. The essential step, from a molecular biology standpoint, of ex vivo gene therapy is the proper introduction of the nucleotide sequence encoding the desired protein into the target cell. The introduction of the desired gene is generally accomplished by the use of viral vectors, although other methods are also applicable. Two primary types of viral vectors are frequently used for the introduction of the foreign gene into the target cell: (1) retroviral vectors and (2) adeno-associated viruses (AAVs). The choice of an appropriate retroviral vector is dependent on the existence of an appropriate viral receptor on the target cell. A variety of retroviral vectors appropriate for the transduction of a variety of cell lines have been described in the literature. The use of retroviral vectors is generally accomplished by the use of "packaging cells" which permit the production of high titers of replication-defective recombinant virus free of wild-type virus. [Miller, Human Gene Therapy, volume 1, number 5 (1990)]. Adeno-associated viruses (AAVs) may also be used to efficiently transfer genes in vitro. Production of high titers of replication-defective recombinant virus has been described. [Flotte et al. Gene Therapy 2:29 (1995); Current Topics in Microbiol. Immunol 158:92 (1992), Kotin PNAS(USA) 87:2211 (1990)].

In vivo gene therapy generally describes the introduction of a nucleotide sequence into an animal in need of therapy without previous isolation of a sample of target tissue or cells. In vivo therapy generally results in foreign nucleotide sequences being transferred to the nucleus of cells of an animal without subsequent integration into the genome. Adenoviral vectors are frequently used to deliver DNA into the cells because these viruses are capable of infecting non-dividing cells and expressing foreign genes. Replication defective adenoviruses lacking portions of the E1 region of the viral genome can be propagated by growth in cells engineered to express the E1 genes (Shenk, Cell 16:683 (1979)). Subsequent studies have indicated that replication defective adenoviruses carrying foreign DNA sequences could infect non dividing cells. Once inside the cells, the foreign DNA has been shown to express active protein [Becker et al., in "Protein Expression in Animal Cells" Methods of Enzymology", (1995)}.

The treatment of human disease by the implementation of gene therapy approaches requires careful design. In particular, certain disease states are associated with defective intracellular post-translational processing mechanisms. Thus, a potential gene therapy approach to such diseases requires either (1) replacing or supplying the defective enzyme or enzymes responsible for post-translational processing of therapeutic proteins or (2) providing a synthetic analog of the native protein which is fully active yet not requiring post-translational processing. The present invention provides an application of the latter approach. Secondly, gene expression is generally a well regulated system in nature. Consequently, constituitive expression of the gene therapy nucleotide sequence is frequently unacceptable.

Type I diabetes mellitus is associated with an inability of the body to produce functional insulin due to a lack of sufficient pancreatic beta cells. In Type II diabetes, although the individual is capable of producing the insulin precursor proinsulin, the body sometimes lacks the ability to fully process proinsulin to insulin. The process of insulin biosynthesis in pancreatic beta cells is well established. See e.g. Steiner D.F (1990) Handbook of Experimental Pharmacology Vol. 92- Insulin, pp. 67-92, and Steiner, D.F. Ch. 1, The Biosynthesis of Biologically Active Peptides: a Perspectivive in Peptide Biosynthesis and Processing, (1991) pp. 1-15, L.D. Fricker, ed., CRC Press, NY. In biosynthesis of insulin in the animal, the insulin molecule is not immediately exported following translation but is rather stored in a hexameric zinc crystalline form in the pancreatic beta cells and released upon stimulation of the insulin receptor by extracelluar glucose. Consequently, effective gene therapy of diabetes mellitus should incorporate a mechanism of regulatory control in response to serum glucose levels.

The in vivo biosynthesis of insulin involves two proteolytic cleavages of the human proinsulin (hPI) structure. For convenience, these enzymes are termed endopeptidase 1 and endopeptidase 2. [Alternative nomenclature has been advanced by Hutton (Hutton, J.C. (1994) Diabetologica 37(suppl.2):S45-S56) and Halban (Halban, P.A. (1994) Diabetologia 37(suppl.2):S65-S72), but for purposes of this discussion the present designation is sufficient.] Endopeptidase 1 cleaves the hPI structure at the C/A junction resulting in a split form of proinsulin. The Arg-Arg residues at the C/A junction are then removed by carboxypeptidase H. The des(31-32)hPI split form of proinsulin is then the preferred substrate of endopeptidase 2. Since the compounds of Formula 1 do not possess the Arg-Arg structure characteristic of the C/A junction of native human proinsulin (hPI) they are not preferred substrates of endopeptidase 1 and although the B/C junction structure of native hPI remains intact, the failure of the cell to cleave at the C/A junction disfavors proteolysis at the B/C junction of the compounds of Formula 1. Consequently, because the compounds of Formula 1 are substrates neither of the initial processing enzyme (endopeptidase 1) nor the secondary processing enzyme (endopeptidase 2), their single chain structure remains intact.

Nucleotides encoding the compounds of Formula 1 generally are useful in the treatment of diabetes mellitus via gene therapy protocols. Thus nucleotide sequences encoding the peptides of the present invention are useful as therapeutic agents in the treatment of Type I diabetes where the nucleotide sequences are incorporated into a vector comprising (1) a mammalian regulatory region (enhancer and promoter) responsive to glucose with a mechanism of action analogous to the mammalian preproinsulin regulatory region, (2) a hybrid nucleotide fragment comprised of a Kozak consensus sequence linked to the sequence encoding the compounds of the present invention and (3) an intron and polyadenylation signal.

Type II diabetes is generally considered to result from the afflicted individual lacking the ability to process preproinsulin to insulin. As stated above, the compounds of the present invention do not require post-translational processing. Thus the incorporation of the nucleotide sequences encoding compounds of the present invention are superior to incorporation of preproinsulin or proinsulin which would not work in such an instance. Using gene knock-out technology, the native preproinsulin gene on chromosome 11 of beta cells in the islets of Langerhans of type II diabetics could be replaced with the nucleotide sequences encoding the compounds of the present invention. The newly replaced gene would have the advantage of use of the native transcriptional control sequences and would thus be regulated in the same manner as the endogenous gene.

In the preferred practice of the invention as exemplified herein, a nucleotide sequence encoding the human preproinsulin signal peptide is linked to a DNA sequence encoding des(31-53)hPI and inserted into a eukaryotic expression vector capable of integration into the target cell line. This eukaryotic expression vector is transfected into cells responsive to changes in the external glucose concentration within conventional physiological limits. These cells are then implanted into an individual suffering from diabetes resulting in extracellular secretion of the des(31-53)hPI analog in response to physiological modulations of blood glucose levels in a mechanism analogous to native beta cells.

### A) Constructing A Synthetic Or Semi-Synthetic Nucleotide Encoding The Compound Of The Formula 1

The coding sequence for the compounds of the Formula 1 may be wholly synthetic, semi-synthetic or the result of modification of the native insulin cDNAs. Synthetic genes, the in vitro or in vivo transcription and translation of which will result in the production of compounds of the Formula 1 may be constructed by techniques well known in the art. Owing to the natural degeneracy of the genetic code, the skilled artisan will recognize that a sizable yet definite number of nucleotide sequences may be constructed which encode the compounds of the Formula 1. The amino acid sequence of human insulin, human proinsulin and human preproinsulin are well known in the art.

A synthetic DNA sequence encoding the single-chain insulin analog of Formula 1 may be prepared by techniques well known in the art in substantial accordance with the teachings of Brown, et al. (1979) Methods in Enzymology, Academic Press, N.Y., Vol. 68, pgs. 109-151. The DNA sequence may be generated using conventional DNA synthesizing apparatus such as an Applied Biosystems Model 380A or 380B DNA synthesizer (commercially available from Applied Biosystems, Foster City, California). Commercial services are also available for the construction of such nucleotide sequences based on the amino acid sequence.

The synthetic gene may also be constructed by assembly of sequences encoding fractions of plasmids containing sequences for the insulin A chain, insulin B-chain, human proinsulin, and human preproinsulin. Such plasmids are well known in the art. United States Patent No. 4,366,246 (issued December 28, 1982) describes DNA sequences encoding and the recombinant production of human insulin A-chain and B-chain. United States patent No. 4,431,740 (issued February 14, 1984) describes cDNA encoding human proinsulin and human preproinsulin.

In the preferred practice of the invention as exemplified herein a synthetic gene is assembled comprising a DNA sequence encoding the native human preproinsulin signal peptide followed by a DNA sequence encoding a compound of the Formula 1 having the DNA sequence:
ATG GCC CTG TGG ATG CGC CTC CTG CCC CTG CTG GCG CTG CTG GCC CTG TGG GGA CCT GAC CCA GCC GCA GCC TTT GTT AAC CAA CAC CTG TGC GGC TCC CAC CTG GTG GAA GCT CTG TAC CTG GTG TGC GGT GAA CGT GGC TTC TTC TAC ACC CCG AAG ACG CAG CCG CTG GCC CTC GAG GGT TCC CTG CAG AAG CGT GGC ATT GTG GAA CAA TGC TGT ACC AGC ATC TGC TCC CTG TAC CAG CTG GAG AAC TAC TGC AAC TAG,
which results in the expression of compound of Formula 1 (des)31-53)hPI with the native human preproinsulin signal peptide sequence, said compound having the amino acid sequence:
Met Ala Leu Trp Met Arg Leu Leu Pro Leu Leu Ala Leu Leu Ala Leu Trp Gly Pro Asp Pro Ala Ala Ala PHE VAL ASN GLN HIS LEU CYS GLY SER HIS LEU VAL GLU ALA LEU TYR LEU VAL CYS GLY GLU ARG GLY PHE PHE TYR THR PRO LYS THR GLN PRO LEU ALA LEU GLU GLY SER LEU GLN LYS ARG GLY ILE VAL GLU GLN CYS CYS THR SER ILE CYS SER LEU TYR GLN LEU GLU ASN TYR CYS ASN

### B) INTEGRATING THE SYNTHETIC GENE INTO A VECTOR IN A MANNER SUITABLE FOR THE EXPRESSION OF THE COMPOUND OF FORMULA 1

In gene therapy, there are alternative means for introducing the genetic information necessary into the target cell. Retroviral Vectors: The use of retroviral vectors allows efficient introduction of genetic information into large numbers of cells. Retroviral vectors, especially those derived from the Maloney murine leukemia virus (M-MLV), are particularly useful in the integration of foreign genetic material into the host chromosome. In the construction of such vectors, the nucleotide sequence encoding the desired compound of Formula 1 is inserted with its control region into the region of the retroviral structural gene region of the retrovirus (e.g. the gag, pol, and env genes).

Retroviral regulatory elements (enhancer and promoters) are capable of conducting efficient expression of the transduced genes in a wide variety of target cell types. However, additional regulatory sequences may be added to achieve tissue specific expression by the use of tissue specific enhancer and promoter elements.

Alternatively adenoviral vectors may be employed. Jones and Shenk (1979) Cell 16:683, demonstrated that replication deficient adenoviruses containing deletions of the E1 region of the viral genome could be propagated in cells engineered to express the E1 genes. Typical constructions of adenoviral gene therapy vectors are constructed by insertion of the foreign DNA into the E1A-E1B and E3 regions of the viral genome. Expression of the foreign DNA sequence is generally under control of the E1A promoter, the major late promoter (MLP) and associated sequences, the E3 promoter.

Other viral vectors are known for their utility in the introduction of foreign nucleotide sequences for gene therapy applications. For example, derivatives of adeno-associated virus (AAV), herpes virus and vaccinia virus vectors have been demonstrated to have utility in gene therapy.

As an alternative to viral introduction utilizes receptor-mediated methods of gene transfer. In such instances, a complex is formed between the target tissue receptor and the plasmid DNA and polypeptides and the receptor on the cell surface. The plasmid DNA is taken up by the cell in endocytotic mediated transfer. The plasmid DNA may then be released from the vesicle after incorporation into the cell.

Construction of suitable vectors containing the desired coding and control sequences may be constructed by standard ligation techniques. Isolated plasmids or nucleotide fragments are cleaved, tailored, and religated in the manner necesary to achieve the plasmids required. To effect the expression of the compounds of Formula 1, one inserts a nucleotide sequence encoding the compound into an appropriate recombinant nucleotide expression vector through the use of appropriate restriction endonucleases. The nucleotide sequence encoding the compound of Formula 1 is designed to possess restriction endonuclease cleavage sites at either end of the transcript to facilitate isolation from and integration into these amplification and expression plasmids. The coding sequence may be readily modified by the use of synthetic linkers to facilitate the incorporation of the coding sequence into the desired cloning vectors by techniques well known in the art. The particular endonucleases employed will be dictated by the restriction endonuclease cleavage pattern of the parent expression vector to be employed. Restriction sites are chosen so as to properly orient the coding sequence such that it is properly associated with the promoter and ribosome binding site of the expression vector, both of which are functional in the host cell in which the compound of the Formula 1 is to be expressed.

In general, plasmid vectors containing promoters and control sequences which are derived from species compatible with the host cell are used. The vector ordinarily carries a replication site as well as marker sequences which are capable of providing phenotypic selection in transformed cells.

In the preferred practice of the invention as exemplified herein, the synthetic gene identified above as Sequence ID No. 3 is assembled from two smaller DNA sequences. The final segment is designed to possess a HindIII restriction site at the 5' end and a BamHI restriction site at the 3' end. The segment is inserted into the MCS (Multiple Cloning Site) of the 5400 bp eukaryotic expression vector pcDNA3 (commercially available from Invitrogen, 3985B Sorrento Valley Blvd., San Diego, CA 92121). Expression of the des(31-53)hPI coding sequence is under control of the CMV immediate early promoter; whereas, the neomycin resistance gene is controlled by the SV40 early promoter.

### Target Cell Selection:

Because the compounds of the present invention do not require post-translational processing mechanisms, such nucleotide sequences encoding these compounds may be incorporated into target cells which do not possess the preproinsulin and proinsulin processing enzymes, i.e. non-beta cells. Secondly, the compounds of the present invention are smaller than preproinsulin and proinsulin and may be more stable in cell lines which do not naturally produce insulin. The techniques of transforming mammalian cells with the aforementioned vectors are well known in the art and may be found in such general references as Maniatis, et al. (1989) Molecular Cloning: A Laboratory Manual, Cold Spring Harbor Press, Cold Spring Harbor Laboratory, Cold Spring Harbor, New York or Current Protocols in Molecular Biology, Vol. 1, (1988), Wiley Interscience, and supplements.

As previously indicated, it is preferred that the secretion of the compound of Formula 1 from the host cell should be under glucose regulatory control. Cells naturally responsive to glucose are preferred as host cells for gene therapy. For example, beta islet cells are naturally responsive to external glucose levels. In the preferred practice of the invention as exemplified herein, the preferred host cell line for ex vivo implantation is the mouse pancreatic beta cell line, βTC6-F7 (Knaack, et al (1994) Diabetes 43:1413-1417).

Constituitive production of insulin is generally not preferred. However, analogs of the compound of Formula 1 having lower insulin activity may be constituitively expressed to maintain a therapeutically effective basal level of circulating insulin activity.

### FORMULATIONS

Compounds of Formula 1, preferably in the form of a pharmaceutically acceptable salt, can be formulated for oral or parenteral administration for the therapeutic or prophylactic treatment of diabetes mellitus and/or non-insulin dependent diabetes mellitus (NIDDM).

For example, compounds of the Formula 1 can be admixed with conventional pharmaceutical carriers and excipients and used in the form of tablets, capsules, elixirs, suspensions, syrups, wafers and the like. The compositions comprising IGF-1 analog compounds will contain from about 0.1 to 90% by weight of the active compound, and more generally from about 10 to 30%. The compositions may contain common carriers and excipients such as corn starch or gelatin, lactose, sucrose, microcrystalline cellulose, kaolin, mannitol, dicalcium phosphate, sodium chloride, and alginic acid. Finkenaur (United States Patent No. 4,179,497) describes an aqueous medicinal composition comprising IGF-1 and a water-soluble polysaccharide to stabilize the peptide against loss of biological activity. This water soluble polysaccharide may be, e.g. hydroxypropyl cellulose, methylcellulose, hydroxyethyl cellulose or hydroxypropyl methylcellulose.

Disintegrators commonly used in the formulations of this invention include croscarmellose, microcrystalline cellulose, corn starch, sodium starch, glycolate and alginic acid. Tablet binders that can be included are acacia, methyl cellulose, sodium carboxymethylcellulose, polyvinylpyrrolidone (Povidone), hydroxypropyl methylcellulose, sucrose, starch and ethylcellulose. Lubricants that can be used include magnesium stearate or other metallic stearates, stearic acid, silicone fluid, talc, waxes, oils and colloidal silica. Flavoring agents such as peppermint, oil of wintergreen, cherry flavoring, or the like can also be used. It may be desirable to add a coloring agent to make the dosage form more attractive in appearance or to help identify the product. For intravenous (IV) use, a water soluble form of compounds of the Formula 1 can be dissolved in one of the commonly used intravenous fluids and administered by infusion. Such fluids, for example, physiological saline, Ringer's solution or 5% dextrose solution can be used. For intramuscular preparations, a sterile formulation of a suitable soluble salt form of the compounds of the Formula 1, for example the hydrochloride salt, can be dissolved and administered in a pharmaceutical diluent such as pyrogen-free water (distilled), physiological saline or 5% glucose solution. A suitable insoluble form of the compound may be prepared and administered as a suspension in an aqueous base or a pharmaceutically acceptable oil base, e.g. an ester of a long chain fatty acid such as ethyl oleate.

In some therapeutic applications, it may be desirable to achieve a prolonged release of the compound. Methods of incorporating the compounds of Formula 1 into such delayed release formulations are well known in the art and may be found in general texts on the subject. A preferred time release formulation of the compounds of the present invention includes the use of poly(alkylcyanoacrylate) particles, commonly known as nanoparticles, as carriers for the compound. The use of nanoparticles is described in Grangier, J.L., et al "Nanoparticles as carriers for growth hormone releasing factor" (1991) J. of Controlled Release 15:3-13. Another preferred formulation of the compounds of the present invention is the use of transdermal patches to provide continuous or discontinuous infusion of the compounds of the present invention in controlled amounts. The construction and use of transdermal patches for the delivery of pharmaceutical agents is well known in the art. Such patches may be constructed for continuous, pulsatile, or on demand delivery of pharmaceutical agents.

Alternatively, the unit dosage form of the compound can be a solution of the compound, preferably in its salt form, in a suitable diluent in sterile hermetically sealed ampoules. The concentration of the compound in the unit dosage may vary, e.g. from about 1% to about 50% depending on the particular form of the compound and its solubility and the dose desired by the physician.

In practicing this method, compounds of the Formula 1 can be administered in a single daily dose or in multiple doses per day. The treatment regime may require administration over extended periods of time. The amount per administered dose or the total amount administered will depend on such factors as the nature and severity of the disease, the age and general health of the patient and the tolerance of the patient to the compound. The method comprises administering to the organism an effective amount the compound in a dose between about 1 and 1000 µg/kg. A preferred dose is from about 10 to 100 µg/kg of active compound. A typical daily dose for an adult human is from about 0.5 to 50 mg.

The pharmaceutical formulations comprising the compounds of Formula 1 described above may be administered to an organism for therapeutic effects. In a method for treating diabetes mellitus and non-insulin dependent diabetes mellitus it is preferred to administer to the organism an effective amount of hybrid insulin/IGF-1 analog in a dose between about 1 and 1000 µg/kg. A preferred dose is from about 10 to 100 µg/kg of active compound. A typical daily dose for an adult human is from about 0.5 to 50 mg.

### Example 1. Solid Phase Peptide Synthesis of bCa

The synthesis of this material (0.5 mMole scale) began with 0.38 g of Boc-Asn-PAM resin. The peptide was synthesized by solid-phase methodology utilizing an Applied Biosystems 430A peptide synthesizer (commercially available from Applied Biosystems, Foster City, California) and synthesis cycles supplied by Applied Biosystems. Boc amino acids and other reagents were supplied by Applied Biosystems and other commercial sources. Boc-His-BOM was obtained from Bachem California, Torrance, California. Sequential Boc chemistry using double coupling and acetic anhydride capping were applied to the desired Boc-amino acid-4-(oxymethyl)-phenylacetamidomethyl [PAM] resin. Asparagine, glutamine, histidine and arginine were coupled using preformed hydroxy benzotriazole esters. All others were made using preformed symmetrical anhydrides with docyclocarbodiimide (DCC).

The following side chain protection is used:
- Arg,: tosyl
- Cys,: 4-methylbenzyl
- Glu,: cyclohexyl
- His,: benzyloxymethyl (BOM)
- Lys,: 2-chlorobenzyloxycarbonyl
- Ser,: benzyl
- Thr,: benzyl
- Tyr,: 2-bromobenzyloxycarbonyl

Boc deprotection was accomplished with TFA in methylene chloride. Following completion of the synthesis the peptide was deprotected and cleaved from the resin with 20 ml of anhydrous HF containing 10% meta-cresol. Cleavage of the side chain protecting groups and of the peptide from the resin were carried out at zero degrees centigrade for one hour. After removal of the HF by vacuum distillation, the peptide/resin was washed with ethyl ether and vacuum dried.

### Example 2. Sulfitolysis of bCa

The peptide was solubilized in 100 ml of 8M guanidine HCl containing 0.2M tris, 0.1M sodium tetrathionate, and 0.1M sodium sulfite followed by filtration. The sulfitolysis reaction (pH 8.7) was maintained at room temperature for three hours, then dialyzed against 0.05M ammonium bicarbonate at 4°C. The pH was lowered to 3.4 at 4°C for one half hour and the resulting isoelectric precipitate was collected by centrifugation.

The precipitate was dissolved in 0.1M ammonium phosphate monobasic, pH 7, and purified on a 2.54 x 30 cm DuPont Zorbax® C8 column using a linear gradient of a acetonitrile in 0.1M ammonium phosphate, pH 7. The appropriate sample pool was rechromatographed on a 2.21 x 25 cm Zorbax® C8 column using a linear gradient of acetonitrile in 0.1M ammonium bicarbonate. Appropriate fractions were combined and lyophilized on the basis of analytical HPLC.

### Example 3. Folding/Disulfide Bond Formation

12.5 mg of the lyophilized bCa S-sulfonated derivative from Example 2 were dissolved in 25 ml of 20mM glycine, pH 9, containing 25 µl of 0.5M cysteine. After seven hours at 4°C, the reaction was quenched by acidification.

### Example 4. Purification of Folded bCa

The folded peptide from Example 3 was purified by multiple analytical runs (0.46 X 25 centimeter Vydac® C18 column eluted with a linear gradient of acetonitrile in 0.1% aqueous TFA). The major peak from each run was combined and lyophilized to yield approximately 0.5 mg of purified protein.

The structure was verified by FAB-MS and amino acid analysis. Results of FAB/MS gave a molecular weight of 7038 (theory 7036). Amino acid composition was as follows based on aspartic acid as unity (theoretical values in parentheses): Asp 3.0(3); Thr 3.5(4); Ser 4.8(6); Glu 7.8(8); Pro 1.7 (2); Gly 7.7(6); Ala 2.0(2); half-Cys 4.8(6); Val 3.5(4); Ile 1.7(2); Leu 5.8(6); Tyr 4.4(5); Phe 2.7(3); His 2.0(2); Lys 1.0(1); Arg 2.8(3).

### Example 5. IGF-1 and Insulin Assay Procedure

Measurements of insulin and IGF-1 activities were accomplished in substantial accordance with the teaching of Gruppuso, et al. (1988) J. of Clin. Endocrin. and Metab. 67:194-197. Briefly, 30-50 µg of human placental membrane protein was incubated with approximately 10 femtomoles of [¹²⁵I] ligand in a final volume of 500 µl of 100 mM HEPES, pH 7.8, 120mM NaCl, 5mM KCl, 1.2mM MgSO₄, 8mM glucose and 0.25% BSA for 24 hours at 4°C. Membranes were collected on glass fiber filters pre-treated with 0.1% polyethyleneimine by using a cell harvester (Skatron, Lier, Norway). Binding data were analyzed by fitting displacement curves to a four parameter model employing Prefit/Allfit® software for the determination of EC₅₀ values.

### Example 6. Recombinant Preparation of des(31-53)hPI

The synthetic DNA sequence identified herein as Sequence ID No.3 was prepared by techniques well known in the art in substantial accordance with the teachings of Brown, et al. (1979) Methods in Enzymology, Academic Press, N.Y., Vol. 68, pgs. 109-151. The DNA sequence was be generated using conventional DNA synthesizing apparatus such as an Applied Biosystems Model 380A or 380B DNA synthesizer (commercially available from Applied Biosystems, Foster City, California).

About 15mg of plasmid pCZR126S (the construction of which is may be found in Belagaje, et al. United States Patent No. 5,304,473 issued April 4, 1994) was suspended in 20µl of 10X NdeI buffer, 5m1 of NdeI restriction enzyme (40 units) and 175µl of water, gently mixed and incubated at 37°C for 2 hours. After the incubation, the DNA was precipitated with three volumes of ethanol as above, dried and then resuspended in 20µ1 of 10X BamHI buffer, 2.5µ1 of BamHI restriction enzyme (25 units) and 178µ1 of water. After gentle mixing, the reaction was incubated at 37°C for 2 hours. The DNA was again precipitated with three volumes of ethanol and electrophoresed on a 1% low melting agarose gel. The larger fragment corresponding to the vector DNA was sliced from this gel and the DNA was recovered. After precipitation and drying the vector DNA was stored at 4°C in 35µl of 10mM tris-HCl pH 8.0.

About 2.5 µl of the vector DNA was mixed with 12 µl of the purified Met-Arg-Met-des(31-53)hPI gene fragment from above, 4 µl of 10mN ATP, 0.5 µl of 1M dithiothreitol, 5 µl of 10X ligase buffer (500mM tris-HCl, pH 7.6, 100mM MgC1₂), 26 µl of water and 0.5 µl of T4 DNA ligase (Pharmacia, Inc., 800 Centennial Avenue, Piscataway, N.J. 08854, 3.5 units). The reaction was incubated at 4°C for 16 hours. The ligated mixture was diluted with 50ml of 10mM tris-HCl (pH 7.6) and 3 µl of lM CaC1₂ and then subsequently transformed into E. coli K12 RV308. The cells were plated on T4 agar plates supplemented with 5 µg/ml tetracycline and incubated overnight at 32°C.

Plasmids from 3 mL cultures were isolated from the tetracycline resistant colonies by the rapid alkaline extraction procedure described in Molecular Cloning: A Laboratory Manual, (1982) edited by Maniatis, T., Fritsch, E.F., and Sambrook, J., Cold Spring Harbor Publications, New York, pgs.368-369. The presence of the correct Met-Arg-Met-des(31-53)hPI gene fragment was determined by the miniscreen procedure according to the teaching of Birnboim, H.C., Edoly, J. (1979) Nucleic Acids, Res. 1, 1513-1523, using polyacrylamide gel elctrophoresis to analyze the XbaI/BamHi digested fragment. Those plasmids with the correct size inserts were selected by amplification and purification. The expression plasmid containing the Met-Arg-Met-des(31-53)hPI gene is called pRB284.

Production of cells for extraction and purification of recombinant Met-Arg-Met-des(31-53)hPI was accomplished using a BioFlo benchtop fermenter (commercially available from New Brunswick Scientific Co., Inc., P.O. Box 986, 44 Talmadge Road, Edison, NJ 08817). Five liters of 2X TY broth containing 5µg/ml tetracycline (obtained from Sigma Chemical Co.) plus 1.0 ml of antifoam SAG 5693 (commercially available from Union Carbide, Specialty Chemical Division, Danbury, CT 06817-0001) was inoculated with 100 ml of bacterial culture of E. coli K12 RV 308 cells containing the pRB284 plasmid were grown overnight at 30°C. Cells were grown at 32°C until the end of the exponential growth phase. Next, glucose and casein amino acids were added to concentrations of 0.2% and 0.1% respectively and the temperature shifted to 42°C to induce protein synthesis. The cells were harvested from the growth medium two hours post-induction by centrifugation at 500 x g for 10 minutes at 4°C. The supernatant was discarded and the pellet was washed once with ice cold TE buffer (10mM Tris-HCl, pH 8.0, 1 mM EDTA).

Expression and accumulation of Met-Arg-Met-des(31-53)hPI was determined by visualization of total cell protein following separation in a 10-20% polyacrylamide pore gradient gel in substantial accordance with the teachings of Laemmli, U.K. (1970) Nature (London), 227:680-685. Pelleted cells were lysed by the addition of modified sample buffer (0.125M Tris-HCl, pH=6.8, 2%SDS, 30% glycerol, 1M 2-mercaptoethanol, 6M urea) and boiled for 5 minutes prior to loading. Bands were detected by staining with Coomassie Blue and quantitated by scanning.

Specific identification of Met-Arg-Met-des(31-53)hPI was determined by Western Blot analysis in substantial accordance with the teaching of Johnson, D.A., et al., (1984) Gene Anal. Tech. Vol. 1, pgs.3-8, using goat anti-HPI which recognizes the C-chain, followed by addition of a biotinylated second antibody (donkey anti-goat IgG) and visualization with the Vectastain™ protein detection kit (commercially available from Vector Laboratories, Inc., 30 Ingold Rd., Burlingame, CA 94010) in substantial accordance with the directions supplied by the vendor.

The frozen homogenate, approximately 2 liters, from a 10 liter E. coli fermention of Met-Arg-Met-des(31-53)hPI were thawed and centrifued at 4°C at 9000 rpm (13,700 x g) for 9-16 hours. The supernatants were discarded and the four pellets were kept frozen and individually processed through the following steps of solubilization and desalting. A fraction of the product was additionally processed through the follwing steps of RP-HPLC and lyophilization.

The pellet was dissolved in 300 ml of 7M guanidine HCl. A Tissuemizer (commercially available from Tekar) was used to completely disperse the pellet.

The sample was recentrifuged at 9000 rpm (13,700 x g) for 30 minutes at 4°C. The pellet was discarded.

Tris, Na₂SO₃ and Na₂S₄O₆ were added to the supernatant obain a final concentration of 0.1M, 0.25 M and 0.1 M respectively. The sample was S-sulfonated by stirring vigorously for approximately 30 minutes at room temperature.

The S-sulfonated sample was solvent exchanged into 7M deionized urea, 0.02M tris, pH 7.9, using a 5 x 215 cm column packed with Pharmacia G10 Sephadex®.

The main stream pool was purified by loading onto a 5 x 55 cm column packed with Pharmacia Fast Flow Q® using a linear gradient of 0 to 1.0 M in sodium chloride in 7M deionized urea, 0.02M tris, pH 7.9 at 4°C. The appropriate fractions were pooled and desalted over a 3.7 x 100 cm column of Pharmacia G25C Sephadex® using 0.05M ammoium bicarbonate at 4°C

After addition of acetonitrile to the desalted pool to achieve 10% v/v, the sample was purified over a 5 x 60 cm C18 reverse phase column using a linear gradient of acetonitrile in 0.1M ammonium bicarbonate. Appropriate fractions were pooled and lyophilized.

646 mg of Met-Arg-Met-des(31-53)hPI (S-sulfonated) were dissolved in 137 ml of 0.2M CNBr in 70% formic acid. The reaction was allowed to proceed at room temperature in the dark for 3 hours.

160 ml of purified water were added to the sample which precipitated the product. The sample was kept frozen then thawed and centrifuged at 2000 rpm (approximately 3000 x g) for one hour at 4°C. The supernatant was discarded.

The pellet was disulfide bond folded by dissolving in 120 ml of 20mM glycine 0.5mM cysteine, pH 10.6, at 4°C. The reaction was quenched after 24 hours by lowering the pH to 3.4 using phosphoric acid.

After addition of acetonitrile to the product to achieve a 10% v/v, the sample was purified over a 2.12 x 25 cm DuPont Zorbax® C8 reverse phase column using a linear gradient of acetonitrile in 0.1M pH 2.3 monobasic sodium phosphate.

Appropriate fractions were pooled and diluted 2 fold with purified water and additionally purified and desalted using a 2.12 x 25 cm Vydac® C18 reverse phase column and a linear gradient of acetonitrile in 0.1% TFA. Selected fractions of des(31-53)hPI were pooled and lyophilized.

The structure was verified by FAB-MS and amino acid analysis. Results of FAB/MS gave a molecular weight of 7111.7 (theory 7111.2). Amino acid composition was as follows based on aspartic acid as unity (theoretical values in parentheses): Asp 3.0(3); Thr 2.8(3); Ser 3.4(4); Glu 10.0(10); Pro 1.9(2); Gly 5.0(5); Ala 2.0(2); half-Cys 4.6(6); Val 3.4(4); Ile 1.5(2); Leu 8.9(9); Tyr 3.7(4); Phe 2.9(3); His 2.7(2); Lys 2.0(2); Arg 1.9(2).

### EXAMPLE 7. Gene Therapy Vector Construction

The synthetic DNA sequences utilized herein may be prepared by techniques well known in the art in substantial accordance with the teachings of Brown, et al. (1979) Methods in Enzymology, Academic Press, N.Y., Vol. 68, pgs. 109-151. The DNA sequences are generated using conventional DNA synthesizing apparatus such as an Applied Biosystems Model 380A or 380B DNA synthesizer (commercially available from Applied Biosystems, Foster City, California).

A recombinant DNA sequence encoding the human preproinsulin signal peptide followed by DNA encoding des(31-53)hPI analog is constructed in two sections. A first section encoding the signal peptide and the 5' end of the B-chain is synthesized by conventional DNA synthesis techniques. This 115 base pair DNA fragment is designed to incorporate a HindIII site at the 5' end and a DraIII site at the 3' end. The sequence of this HindIII-DraIII DNA fragment is as follows:
AGCTTCCACC ATG GCC CTG TGG ATG CGC CTC CTG CCC CTG CTG GCG CTG CTG GCC CTG TGG GGA CCT GAC CCA GCC GCA GCC TTT GTT AAC CAA CAC CTG TGC GGC TCC CAC CTG.
A second DNA fragment encoding the remainder of the human insulin B-chain, amino acids 54-65 of the human insulin C-peptide and the human insulin A-chain is constructed as a 171 base pair DNA sequence designed to incorporate a DraIII restriction endonuclease cleavage site at its 5' end and a BamHI restriction endonuclease cleavage site at its 3' end. The DNA sequence of this DraIII-BamHI fragment is as follows:
GTG GAA GCT CTG TAC CTG GTG TGC GGT GAA CGT GGC TTC TTC TAC ACC CCG AAG ACG CAG CCG CTG GCC CTC GAG GGT TCC CTG CAG AAG CGT GGC ATT GTG GAA CAA TGC TGT ACC AGC ATC TGC TCC CTG TAC CAG CTG GAG AAC TAC TGC AAC TAG.
The HindIII-DraIII fragment and the DraIII-BamHI fragment are ligated resulting in a HindIII-BamHI fragment encoding the native human preproinsulin signal peptide, the human insulin B-chain, amino acids 54-65 of the human proinsulin C-peptide, and the human insulin A-chain.

### Example 8. Vector Construction

This HindIII-BamHI fragment prepared in substantial accordance with the teaching of Example 7 above is inserted between the HindIII and BamHI sites in the MCS (Multiple Cloning Site) of the 5400 bp eukaryotic expression vector pcDNA3 (commercially available from Invitrogen, 3985B Sorrento Valley Blvd., San Diego, CA 92121). The resulting vector is termed phPIdes(31-53). Expression of the des(31-53)hPI coding sequence is under control of the CMV immediate early promoter; whereas, the neomycin resistance gene is controlled by the SV40 early promoter.

### Example 9. Transfection

The recombinant vector phPIdes(31-53) prepared in substantial accordance with Example 8 above is then transfected into the mouse pancreatic beta cell line, βTC6-F7 (Knaack, et al (1994) Diabetes 43:1413-1417, 1994) using standard lipid mediated transfection protocols. (See e.g. Current Protocols in Molecular Biology, reference specifics).

The cells are cultured in Dulbecco's modified Eagle's medium containing 25 mmol/liter glucose and supplemented with 15% horse serum and 2.5% fetal bovine serum at 37°C in 5% CO₂.

### Example 10. Establishment of Monclonal Cell Lines

Forty eight hours after transfection, cells containing phPIdes(31-53) are selected using the antibiotic G418 (Geneticin sulfate). After three to four weeks in medium containing G418, monoclonal cell lines are established which secrete the des(31-53)hPI protein in response to physiological levels of glucose.

Because βTC6-F7 cells release insulin in response to physiological concentrations of glucose, the des(31-53)hPI protein is secreted into the culture media when the cells are grown in the presence of 5-15 mmol/liter glucose. Identification of those cultures producing the des(31-53)hPI protein are identified and quantitated by immunoassay using an antibody which recognizes the junction of the C-A junction of human proinsulin. A polyclonal antibody is commercially available from Linco Research, Inc., St. Charles, MO 63304.

### Example 11. Implantation

The cells prepared in accordance with the teaching of Example 10 are implanted by surgical procedure into an animal suffering from diabetes mellitus type I or II. In response to physiological changes in glucose concentration, the des(31-53)hPI analog is released allowing modulation of serum glucose levels.

## Claims

1. A polypeptide compound of the formula (1):
b-BP-a (1)
wherein:
b is the B-chain of insulin or a functional analog thereof,
a is the A-chain of insulin or a functional analog thereof, and
BP is a Bridging Peptide of from about 10 to about 14 amino acids.

2. The compound of Claim 1 wherein b and a are the naturally occuring B-chain and A-chain of human insulin, respectively.

3. The compound of Claim 2, wherein BP is about 12 amino acids.

4. The compound of Claim 3 consisting essentially of the amino acid sequence:
Phe-Val-Asn-Gln-His-Leu-Cys-Gly-Ser-His-Leu-Val-Glu-Ala-Leu-Tyr-Leu-Val-Cys-Gly-Glu-Arg-Gly-Phe-Phe-Tyr-Thr-Pro-Lys-Thr-Gly-Tyr-Gly-Ser-Ser-Ser-Arg-Arg-Ala-Pro-Gln-Thr-Gly-Ile-Val-Glu-Gln-Cys-Cys-Thr-Ser-Ile-Cys-Ser-Leu-Tyr-Gln-Leu-Glu-Asn-Tyr-Cys-Asn.

5. The compound of Claim 3 consisting essentially of the amino acid sequence:
Phe-Val-Asn-Gln-His-Leu-Cys-Gly-Ser-His-Leu-Val-Glu-Ala-Leu-Tyr-Leu-Val-Cys-Gly-Glu-Arg-Gly-Phe-Phe-Tyr-Thr-Pro-Lys-Thr-Gln-Pro-Leu-Ala-Leu-Glu-Gly-Ser-Leu-Gln-Lys-Arg-Gly-Ile-Val-Glu-Gln-Cys-Cys-Thr-Ser-Ile-Cys-Ser-Leu-Tyr-Gln-Leu-Glu-Asn-Tyr-Cys-Asn.

6. A pharmaceutical formulation comprising, as an active ingredient a polypeptide compound of formula I, or a pharmaceutically acceptable salt thereof, as claimed in any one of Claims 1 to 5, associated with one or more pharmaceutically acceptable carriers thereof.

7. A polypeptide compound of formula I, or a pharmaceutically acceptable salt thereof, as claimed in any one of Claims 1 to 5, for use in treating diabetes.

8. A nucleotide sequence encoding a polypeptide of formula 1, as claimed in any one of Claims 1 to 5.

9. The nucleotide sequence of Claim 8 consisting essentially of the DNA sequence
TTT GTT AAC CAA CAC CTG TGC GGC TCC CAC CTG GTG GAA GCT CTG TAC CTG GTG TGC GGT GAA CGT GGC TTC TTC TAC ACC CCG AAG ACG CAG CCG CTG GCC CTC GAG GGT TCC CTG CAG AAG CGT GGC ATT GTG GAA CAA TGC TGT ACC AGC ATC TGC TCC CTG TAC CAG CTG GAG AAC TAC TGC AAC.

10. A recombinant nucleotide vector comprising a nucleotide sequence as claimed in Claims 8 or 9.

11. A cell line transformed with a recombinant nucleotide vector of Claim 10.

12. The cell line as claimed in Claim 11 for use in implanting the cell line for treating diabetes.
